# EUROPEAN PATENT SPECIFICATION

(11) **EP 2 356 467 B1**
(45) Date of publication and mention of the grant of the patent: **10.07.2013**
(21) Application number: 09768592.9
(22) Date of filing: 11.12.2009
(51) Int. Cl.: G01N 33/86, G01N 33/68, G01N 21/00, G01N 33/487

(54) **DETECTION OF PHYSIOLOGICALLY ACCEPTABLE POLYMER MOLECULES USING NEAR INFRARED SPECTROSCOPY**
NACHWEIS PHYSIOLOGISCH UNBEDENKLICHER POLYMERMOLEKÜLE MITTELS NAHINFRAROT-SPEKTROSKOPIE
DÉTECTION DE MOLÉCULES DE POLYMÈRE ACCEPTABLES PHYSIOLOGIQUEMENT AU MOYEN DE LA SPECTROSCOPIE PROCHE INFRAROUGE

(30) Priority: 11.12.2008 US 121788 P
(43) Date of publication of application: 17.08.2011
(73) Proprietor: Baxter International Inc., Deerfield, IL 60015 (US); Baxter Healthcare SA, 8152 Glattpark (Opfikon) (CH)
(72) Inventor: WEBER, Alfred, A-1210 Vienna (AT); HOEFINGHOFF, Joris, A-1110 Vienna (AT); SIEKMANN, Juergen, A-1210 Vienna (AT); TURECEK, Peter, A-3400 Klosterneuburg (AT)
(74) Representative: Bassett, Richard Simon
(86) International application number: PCT/US2009/067732
(87) International publication number: WO 2010/068906

(56) References cited:
- DUAN WUBIAO ET AL: "Near IR emitting isothiocyanato-substituted fluorophores: their synthesis and bioconjugation to monoclonal antibodies." ORGANIC & BIOMOLECULAR CHEMISTRY 7 JUL 2005, vol. 3, no. 13, 7 July 2005 (2005-07-07), pages 2384-2386, XP002571161 ISSN: 1477-0520
- TUNG C H ET AL: "NOVEL FACTOR XIII PROBES FOR BLOOD COAGULATION IMAGING" CHEMBIOCHEM - A EUROPEAN JOURNAL OF CHEMICAL BIOLOGY, WILEY VCH, WEINHEIM, DE, vol. 4, 1 January 2003 (2003-01-01), pages 897-899, XP008058462 ISSN: 1439-4227
- CHRISTINE H PETTER ET AL: "Near infrared spectroscopy compared to liquid chromatography coupled to mass spectrometry and capillary electrophoresis as a detection tool for peptide reaction monitoring" AMINO ACIDS ; THE FORUM FOR AMINO ACID AND PROTEIN RESEARCH, SPRINGER-VERLAG, VI, vol. 34, no. 4, 20 December 2007 (2007-12-20), pages 605-616, XP019634448 ISSN: 1438-2199
- JARVIS ROGER M ET AL: "Quantification of casein phosphorylation with conformational interpretation using Raman spectroscopy." THE ANALYST OCT 2007, vol. 132, no. 10, October 2007 (2007-10), pages 1053-1060, XP002571162 ISSN: 0003-2654
- MENG FANTAO ET AL: "PEGylation of human serum albumin: Reaction of PEG-phenyl-isothiocyanate with protein" BIOCONJUGATE CHEMISTRY, vol. 19, no. 7, July 2008 (2008-07), pages 1352-1360, XP002571163 ISSN: 1043-1802
- RODRIGUEZ-MARTINEZ JOSE A ET AL: "Stabilization of alpha-Chymotrypsin Upon PEGylation Correlates With Reduced Structural Dynamics" BIOTECHNOLOGY AND BIOENGINEERING, vol. 101, no. 6, 11 June 2008 (2008-06-11) , pages 1142-1149, XP002571164 ISSN: 0006-3592
- SENGONUL MERIH ET AL: "Surface modification of protein nanocontainers and their self-directing character in polymer blends." POLYMER, vol. 48, no. 13, 2007, pages 3632-3640, XP002571165 ISSN: 0032-3861
- RONALD J BERNARDI ET AL: "Immunonanoshells for targeted photothermal ablation in medulloblastoma and glioma: an in vitro evaluation using human cell lines" JOURNAL OF NEURO-ONCOLOGY, KLUWER ACADEMIC PUBLISHERS, BO, vol. 86, no. 2, 6 September 2007 (2007-09-06), pages 165-172, XP019555486 ISSN: 1573-7373
- PHILIP MOLYNEUX: 'Water-Soluble Synthetic Polymers: Properties and Behaviours', vol. 1, part CHAPTERS 3, 4 CRC PRESS page 75, 119
- A Dictionary of Chemistry, Oxford University Press, 6th ed, John Dainwith, definition of "polymer".
- Dictionary of Chemistry, McGraw-Hill, 2nd ed, definition of "polymer".

## Description

### FIELD OF THE INVENTION

The present invention relates, in general, to a method for determining the amount of a physiologically acceptable polymer molecule bound to a protein.

### BACKGROUND OF THE INVENTION

The *in vivo* function of a protein can be improved by binding it to a physiologically acceptable polymer molecule, e.g., polyethylene glycol. In particular, binding a physiologically active protein to a physiologically acceptable polymer molecule can substantially prolong its *in vivo* half-life. For example, U.S. patent 4,970,300 describes that the conjugation of a physiologically acceptable polymer molecule to Factor VIII results in a Factor VIII protein being activatable by thrombin, and having a substantially decreased antigenicity and immunoreactivity and a substantially increased in vivo disappearance time in the bloodstream of a mammal. International patent application WO 94/15625 describes that conjugating Factor VIII to a physiologically acceptable polymer molecule improves the *in vivo* function of Factor VIII (i) by increasing its resistance to *in vivo* hydrolysis and thus prolonging its activity after administration, (ii) by significantly prolonging its circulating life *in vivo* over unmodified protein, and (iii) by increasing its absorption time into the blood stream. Further, improving the *in vivo* function of Factor IX by binding it to physiologically acceptable polymer molecules, in particular poly(ethylene glycol) ("PEG"), has been described in the international patent application WO 94/29370.

However, at present no reliable method for the quantitative determination of physiologically acceptable polymer molecules bound to proteins or nanoparticles is available. Colorimetric methods (Nag et al., Anal Biochem 250:35-43, 1997) are insensitive and allow only an estimation of the content of polymer molecules and exhibit a detection limit of about 1-5 µg PEG. High performance liquid chromatography can detect PEG with a detection limit around 1-5 µg/ml (Kinahan et al., J Chromatogr 565:297-307, 1991); Ryan et al., J Pharm Sci 81:350-352, 1992); Ruddy et al. J Chromatogr B Biomed Appl 657:83-92, 1994). Phase-partitioning can be employed to measure PEG but the assay sensitivity is about 1 µg PEG (Guermant et al., Anal Biochem 230:254-258, 1995). Also, monoclonal antibodies for the determination of PEG concentrations have been disclosed (U.S. Patent 6,617,118), but so far no system is available for the reliable determination of the amount of a physiologically acceptable polymer molecule bound to a protein. Meng et al 2008 Bioconjugate Chem 19: 1352-1360 discloses PEGylated human serum albumin and a method to detect the quenching of intrinsic tryptophan fluorescence by PEG in the conjugates following excitation at 295 nm.

Near Infrared Spectroscopy (NIRVIS) is the measurement of the wavelength and intensity of the absorption of near-infrared light by a sample. NIRVIS measures the oscillation of chemical bonds in the near infrared light and gives valuable information about the structure of molecules (Landau et al., J Agric Food Chem 50:1374-8, 2002). Near infrared light spans a wavelength range of approximately 680-2500 nm (NIR wavenumbers: about 15,000-4,000 cm⁻¹) and is energetic enough to excite overtones and combinations of molecular vibrations to higher energy levels. The near-IR region is often sub-divided into the short (680-1100 nm) and long (1100-2500 nm) near-IR wavelengths, based upon the technology required to analyze light in these wavelength regions (U.S. Patent 7,280,866). Long wavelength near-IR absorptions arise from overtones and combination bands of the molecular vibrations of C--H, N--H and O--H groups. NIRVIS is typically used for quantitative measurement of organic functional groups, especially O-H, N-H, and C=O. Detection limits are typically 0.1% and applications include pharmaceutical, agricultural, polymer, and clinical analysis. The convenient instrumentation of near infrared spectroscopy compared to infrared spectroscopy makes it much more suitable for online monitoring and process control. Duan et al (Org. Biomol. Chem., 2005, 3, 2384-2386) discloses using NIRVIS to determine the degree of labelling of an antibody.

Thus, there exists a need in the art to provide a new system for the quantitative determination of the amount of a physiologically acceptable polymer molecule bound to a protein.

### SUMMARY OF THE INVENTION

The present invention is directed to the discovery that near infrared spectroscopy is useful to measure the degree of polymer conjugate on a protein molecule, and to determine the uniformity of the protein-polymer conjugate resulting from a conjugation reaction.

In one aspect, the invention provides a method for determining the number of water soluble polymer moieties conjugated to a protein or protein complex in a sample, comprising contacting the sample with a light source in the near infrared spectrum and measuring relative absorbance levels over a range of the near-infrared wavelengths, determining the number of polymer molecules conjugated to the protein or protein complex based on wavelength spectra, wherein the wavelength spectra of the sample is compared to a previously calculated standard having a known amount of polymer molecules conjugated to a protein or protein complex. It is contemplated that the NIR spectra is also expressed in wavenumber.

In one embodiment, the contacting further comprises measuring reflected radiation from said sample as a plurality of time-dependant absorbance spectra.

In a related embodiment, the comparison comprises constructing a calibration data matrix from a plurality of near-infrared absorption spectra of known samples and comparing the measured spectroscopy of the sample comprising the polymer-protein conjugate.

The near infrared wavelength is in the range of 680-2500 nm. In another embodiment, the wavelength is from 680 to 1100 nm. In yet another embodiment, the wavelength is from 1100 to 2500 nm. In a still further embodiment, the near infrared spectra are expressed in wavenumbers and the wavenumber is from 4008 cm⁻¹ to 9996 cm⁻¹ (about 2500 to 1000 nm).

In another embodiment, the physiologically-acceptable polymer is polyethylene glycol (PEG). It is contemplated that the PEG is in a range of 3 to 100 kDa, 5 to 60 kDa, 5 to 40 kDa, 5 to 25 kDa, 5 to 15 kDa, or in a range of 5 to 10 kDa.

In a further embodiment, the invention provides that the protein or protein complex is a therapeutic coagulation factor (blood factor) protein or therapeutic coagulation factor protein complex. In one embodiment, the protein or protein complex comprises a blood factor selected for the group consisting of Factor II, Factor III, Factor V, Factor VII, Factor VIII, Factor IX, Factor X, Factor XI, von Willebrand Factor and fibrinogen.

In a related embodiment, the protein is von Willebrand factor. In an additional embodiment, the protein complex comprises von Willebrand Factor and Factor VIII.

In another aspect, the invention contemplates a process for making a composition having a uniform number of water soluble polymer molecules on a protein or protein complex comprising: conjugating the protein to a water soluble molecule to produce a sample of a polymer-protein complex; performing the method described above on the polymer-protein complex; and isolating compounds within the sample having the same number of polymer molecules based on the near infrared absorbance spectra.

### BRIEF DESCRIPTION OF THE DRAWINGS

Figure 1 shows a calibration model to determine the PEGylation reaction progress over time using a multiplicative scatter correction (MSC) and a PLS-algorithm.

Figure 2 shows a calibration model to determine the amount of a polymer molecule on a protein using a chemometric approach.

### DETA1LED DESCRIPTION

The present invention is directed to methods for sensitive quantification of an amount of a physiologically acceptable polymer molecule bound to a protein.

The term "sample" as used herein refers to any sample containing at least one protein bound to at least one physiologically acceptable polymer molecule, such as any fluid or solution originating from a process for preparing pharmaceutical products.

The term "protein" as used herein refers to any protein, protein complex or polypeptide, including recombinant proteins, protein complexes and polypeptides composed of amino acid residues linked via peptide bonds. Proteins may be obtained by isolation of a protein from in vivo, by synthetic methods or obtained via recombinant DNA technology. Synthetic polypeptides can be synthesized, for example, using an automated polypeptide synthesizer. A recombinant protein used according to the present invention may be produced by any method known in the art as described herein below. In one embodiment, the protein is a physiologically active protein, including a therapeutic protein or a biologically active derivative thereof. The term "biologically active derivative" refers to a modification of a protein having substantially the same functional and/or biological properties of the parent protein. The term "protein" typically refers to large polypeptides. The term "peptide" typically refers to short polypeptides. As used herein, polypeptide, protein and peptide are used interchangeably. A "protein complex" refers to a molecule that is comprised of at least one protein bound to at least one other protein. Examples of protein complexes include, but are not limited to, a protein bound to a cofactor or chaperone protein, ligand-receptor complexes and multisubunit proteins such as integrins and other cell surface receptors comprises of multiple protein subunits.

As used herein a "fragment" of a polypeptide refers to any portion of the polypeptide smaller than the full-length polypeptide or protein expression product. Fragments are typically deletion analogs of the full-length polypeptide wherein one or more amino acid residues have been removed from the amino terminus and/or the carboxy terminus of the full-length polypeptide. Accordingly, "fragments" are a subset of deletion analogs described below.

As used herein an "analog" or "derivative" (which may be used interchangeably) refers to a polypeptide substantially similar in structure and having the same biological activity, albeit in certain instances to a differing degree, to a naturally-occurring molecule. Analogs differ in the composition of their amino acid sequences compared to the naturally-occurring polypeptide from which the analog is derived, based on one or more mutations involving (i) deletion of one or more amino acid residues at one or more termini of the polypeptide and/or one or more internal regions of the naturally-occuning polypeptide sequence, (ii) insertion or addition of one or more amino acids at one or more termini (typically an "addition" analog) of the polypeptide and/or one or more internal regions (typically an "insertion" analog) of the naturally-occurring polypeptide sequence or (iii) substitution of one or more amino acids for other amino acids in the naturally-occurring polypeptide sequence. Substitutions can be conservative or non-conservative based on the physico-chemical or functional relatedness of the amino acid that is being replaced and the amino acid replacing it.

In one aspect, an analog exhibits about 70% sequence similarity but less than 100% sequence similarity with a given compound, *e.g.,* a peptide. Such analogs or derivatives are, in one aspect, comprised of non-naturally occurring amino acid residues, including by way of example and not limitation, homoarginine, ornithine, penicillamine, and norvaline, as well as naturally occurring amino acid residues. Such analogs or derivatives are, in another aspect, composed of one or a plurality of D-amino acid residues, or contain non-peptide interlinkages between two or more amino acid residues. The term "derived from" as used herein refers to a polypeptide or peptide sequence that is a modification (including amino acid substitution or deletion) of a wild-type or naturally-occurring polypeptide or peptide sequence and has one or more amino acid substitutions, additions or deletions, such that the derivative sequence shares about 70% but less than 100% sequence similarity to the wild-type or naturally-occurring sequence. In one embodiment, the derivative may be a fragment of a polypeptide, wherein the fragment is substantially homologous (i.e., at least 70%, at least 75%, at least 80%, at least 85%, at least 90%, or at least 95% homologous) over a length of at least 5, 10, 15, 20, 25, 30, 35, 40, 45 or 50 amino acids of the wild-type polypeptide.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection. One example of a useful algorithm is PILEUP, which uses a simplification of the progressive alignment method of Feng & Doolittle, J. Mol. Evol. 35:351-360 (1987) and is similar to the method described by Higgins & Sharp, CABIOS 5:151-153 (1989). Another algorithm useful for generating multiple alignments of sequences is Clustal W (Thompson et al., Nucleic Acids Research 22: 4673-4680 (1994)). An example of algorithm that is suitable for determining percent sequence identity and sequence similarity is the BLAST algorithm (Altschul et a/., J. Mol. Biol. 215:403-410 (1990); Henikoff & Henikoff, Proc. Natl. Acad. Sci. USA 89:10915 (1989); Karlin & Altschul, Proc. Natl. Acad. Sci. USA 90:5873-5787 (1993)). Software for performing BLAST analyses is publicly available through the National Center for Biotechnology Information.

Substitutions are conservative or non-conservative based on the physico-chemical or functional relatedness of the amino acid that is being replaced and the amino acid replacing it. Substitutions of this type are well known in the art. Alternatively, the invention embraces substitutions that are also non-conservative. Exemplary conservative substitutions are described in Lehninger, [Biochemistry, 2nd Edition; Worth Publishers, Inc., New York (1975), pp.71-77] and set out below.

### CONSERVATIVE SUBSTITUTIONS

| **SIDE CHAIN CHARACTERISTIC** | | **AMINO ACID** |
|---|---|---|
| Non-polar (hydrophobic): | | |
| | A. Aliphatic | A L I V P |
| | B. Aromatic | F W |
| | C. Sulfur-containing | M |
| | D. Borderline | G |
| Uncharged-polar: | | |
| | A. Hydroxyl | STY |
| | B. Amides | N Q |
| | C. Sulfhydryl | C |
| | D. Borderline | G |
| Positively charged (basic) | | K R H |
| Negatively charged (acidic) | | D E |

Alternatively, exemplary conservative substitutions are set out immediately below.

### CONSERVATIVE SUBSTITUTIONS II

| **ORIGINAL RESIDUE** | **EXEMPLARY SUBSTITUTION** |
|---|---|
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| Gln (Q) | Asn |
| Glu (E) | Asp |
| His (H) | Asn, Gln, Lys, Arg |
| Ile (I) | Leu, Val, Met, Ala, Phe, |
| Leu (L) | Ile, Val, Met, Ala, Phe |
| Lys (K) | Arg, Gln, Asn |
| Met (M) | Leu, Phe, Ile |
| Phe (F) | Leu, Val, Ile, Ala |
| Pro (P) | Gly |
| Ser (S) | Thr |
| Thr (T) | Ser |
| Trp (W) | Tyr |
| Tyr (Y) | Trp, Phe, Thr, Ser |
| Val (V) | Ile, Leu, Met, Phe, Ala |

As used herein a "variant" refers to a protein or analog thereof that is modified to comprise additional chemical moieties not normally a part of the molecule. Such moieties may improve the molecule's solubility, absorption, biological half-life, etc. The moieties may alternatively decrease the toxicity of the molecule and eliminate or attenuate any undesirable side effect of the molecule, etc. Moieties capable of mediating such effects are disclosed in Remington's Pharmaceutical Sciences (1980). Procedure for coupling such moieties to a molecule are well known in the art. For example, the variant may be a blood clotting factor having a chemical modification which confers a longer half-life in vivo to the protein. In certain aspects, variants are polypeptides that are modified by glycosylation, PEGylation, or polysialylation.

As used herein, "naturally-occurring," as applied to a protein or polypeptide, refers to the fact that the protein can be found in nature. For example, a polypeptide or polynucleotide sequence that is present in an organism (including viruses) that can be isolated from a source in nature and which has not been intentionally modified by man in the laboratory is naturally-occurring. The terms "naturally-occurring" and "wild-type" are used interchangeably throughout.

As used herein, "plasma-detived," as applied to a protein or polypeptide, refers to a naturally-occurring polypeptide or fragment thereof that is found in blood plasma or serum of a subject. A plasma-derived protein may also be a naturally-occurring protein and a wild-type protein.

The term "physiologically acceptable molecule" or "physiologically acceptable polymer" as used herein refers to polymer molecules which are substantially soluble in aqueous solution or may be present in form of a suspension and have substantially no negative impact to mammals upon administration of a polymer-protein conjugate in a pharmaceutically effective amount and can be regarded as biocompatible. In one embodiment, physiologically acceptable molecules comprise from about 2 to about 2000 repeating units. Exemplary physiologically acceptable polymers include, but are not limited to, poly(alkylene glycols) such as polyethylene glycol (PEG), poly(propylene glycol) ("PPG"), copolymers of ethylene glycol and propylene glycol and the like, poly(oxyethylated polyol), poly(olefinic alcohol), poly(vinylpyrrolidone), poly(hydroxyalkylmethacrylamide), poly(hydroxyalkylmethacrylate), poly(saccharides), poly(α-hydroxy acid), poly(vinyl alcohol), polyphosphasphazene, polyoxazoline, poly(N-acryloylmorpholine), poly(alkylene oxide) polymers, poly(maleic acid), poly(DL-alanine), polysaccharides, such as carboxymethylcellulose, dextran, hyaluronic acid and chitin, poly(meth)acrylates, and combinations of any of the foregoing.

The physiologically acceptable polymer molecule is not limited to a particular structure and can be linear (e.g. alkoxy PEG or bifunctional PEG), branched or multi-armed (e.g. forked PEG or PEG attached to a polyol core), dendritic, or with degradable linkages. Moreover, the internal structure of the polymer molecule can be organized in any number of different patterns and can be selected from the group consisting of homopolymer, alternating copolymer, random copolymer, block copolymer, alternating tripolymer, random tripolymer, and block tripolymer.

The term "PEGylated" as used herein refers to a protein, protein complex or polypeptide bound to one or more PEG moieties. The term "PEGylation" as used herein refers to the process of binding one or more PEGs to a protein. In one embodiment, the molecular weight of said PEG is in the range of from 3 to 100 kDa, from 5 to 60 kDA, from 5 to 40 kDa, from 5 to 25 kDa, from 5 to 15 kDa, or from 5 to 10 kDa.

"Pharmaceutical composition" refers to a composition suitable for pharmaceutical use in subject animal, including humans and mammals. A pharmaceutical composition comprises a pharmacologically effective amount of a polymer-polypeptide conjugate and also comprises a pharmaceutically acceptable carrier. A pharmaceutical composition encompasses a composition comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound or conjugate of the present invention and a pharmaceutically acceptable carrier.

"Pharmaceutically acceptable carrier" refers to any of the standard pharmaceutical carriers, buffers, and excipients, such as a phosphate buffered saline solution, 5% aqueous solution of dextrose, and emulsions, such as an oil/water or water/oil emulsion, and various types of wetting agents and/or adjuvants. Suitable pharmaceutical carriers and formulations are described in Remington's Pharmaceutical Sciences, 19th Ed. (Mack Publishing Co., Easton, 1995). Preferred pharmaceutical carriers depend upon the intended mode of administration of the active agent. Typical modes of administration include enteral (*e.g.,* oral) or parenteral (*e.g.,* subcutaneous, intramuscular, intravenous or intraperitoneal injection; or topical, transdermal, or transmucosal administration). A "pharmaceutically acceptable salt" is a salt that can be formulated into a compound or conjugate for pharmaceutical use including, *e.g.,* metal salts (sodium, potassium, magnesium, calcium, *etc.*) and salts of ammonia or organic amines.

"Pharmaceutically acceptable" refers to a material which is not biologically or otherwise undesirable, *i.e.,* the material may be administered to an individual without causing any undesirable biological effects or interacting in a deleterious manner with any of the components of the composition in which it is contained.

### Near Infrared (NIR)

Near infrared light spans a wavelength range of 700-2500 nm (NIR wavenumbers: about 14,000-3,500 cm⁻¹) and comprises sufficient energy to excite overtones and combinations of molecular vibrations to higher energy levels. Near infrared (NIR) absorptions originate in molecular vibrations, such as fundamental absorptions found in mid-infrared (MIR) band (Raman) spectroscopy, and overtones and combinations found in NIR band (NIR) spectroscopy. Long wavelength near-IR absorptions arise from overtones and combination bands of the molecular vibrations of C--H, N--H and O--H groups. Near infrared spectroscopy (NIRVIS) is typically used for quantitative measurement of organic functional groups, especially O-H, N-H, and C=O.

The near-IR region is often sub-divided into the short (680-1100 nm) and long (1100-2500 nm) near-IR wavelengths, based upon the technology required to analyze light in these wavelength regions (U.S. Patent 7,280,866). NIR spectroscopy may also be divided into transmission spectroscopy of liquids, approximately 750 to 1100 nm, and reflection spectroscopy of powdered materials, approximately 1100 to 2500 nm.

In order to detect different structures in a sample, the NIR spectrum is taken at intervals of length 10 nm or less in order to detect all the different structures in the spectrum, thereby resulting in a multivariate data set. Therefore, a combination of many spectral frequencies is utilized in order to estimate the concentrations of the product constituents, which are resolved into useful data points using chemometric (statistical) methods.

The components and design of NIRVIS instrumentation are similar to UV absorption spectrometers. The light source is usually a tungsten lamp and the detector is usually a PbS solid-state detector. Sample holders can be glass or quartz and typical solvents are CCl₄ and CS₂. Source light is provided from multiple single wavelength sources, such as low coherence superluminescent diodes (SLEDs) at wavelengths in the red/near infrared range (RNIR). Alternatively, the light can be provided from a single broadband source which is appropriately notch filtered. Both wavelengths of light are advantageously directed in a single beam.

In the method according to the present invention, the near infrared absorption spectrum is measured using a wavelength of about 680 to about 2500, in another example at a wavelength of about 680 to about 1100, in a further example using a wavelength of about 1000 to about 2500 nm, and in a still further example from 1100 to about 2500 nm.

In a related embodiment, the NIR absorption is measured according to wavenumbers. The NIR is measured from about 15000 cm⁻¹ to 4000 cm⁻¹, from about 15000 to 9000 cm⁻¹, or from about 9000 to about 4000 cm⁻¹. In one embodiment, the NIR is measured from about 10000 to about 4000 cm⁻¹.

Subsequent to near infrared spectroscopy the amount of polymer per protein molecule is determined using techniques known in the art. For example, chemometric spectroscopy is the term used to describe the direct combination of a spectroscopic measurement with a chemometric data evaluation procedure, which is typically a multivariate statistic method providing a deduction of the qualitative and quantitative properties of a sample from its spectrum (Næs et al., Multivariate Calibration and Classification, NIR Publications, Chichester, 2002). Chemometric methods of analysis are described in Roggo et al., (J Pharm Biomed Anal 44:683-700, 2007). In one embodiment, a multi-linear regression (MLR) or partial least squares (PLS) regression is used for the quantitative determination of individual or combined parameters. Other methods of chemometric analysis include, but are not limited to, principle component analysis (PCA), linear discriminant analysis (LDA), soft independent modeling of class analogy (SIMCA), and discriminant partial least squares (PLS1 and PLS2) regression.

Determining the amount of a physiologically acceptable polymer molecule bound to a protein in a sample is carried out by correlating the near infrared absorption spectrum of said sample with the near infrared absorption spectrum of a sample containing the protein bound to a defined amount of the physiologically acceptable polymer molecule. In one embodiments, the correlation includes the step of providing a calibration curve obtained by measuring the near infrared absorption spectra of samples containing proteins bound to various defined amounts of the physiologically acceptable polymer molecules.

### Proteins and Protein Complexes

Proteins contemplated for use in the compositions include physiologically active protein useful for administration to a subject. In one embodiment, the physiologically active protein is a therapeutic protein. The physiologically active protein, is in one aspect, a protein or any fragment of such that still retains some, substantially all, or all of the therapeutic or biological activity of the protein. In some embodiments, the protein is one that, if not expressed or produced or if substantially reduced in expression or production, would give rise to a disease. Preferably, the protein is derived or obtained from a human.

In various embodiments of the invention, when the physiologically active protein conjugated to a physiologically acceptable polymer is a protein or fragment thereof possessing a biological activity of the protein, the physiologically active protein has an amino acid sequence identical to the amino acid sequence to the corresponding portion of the unconjugated human or mammalian protein. In other embodiments, the physiologically active protein of the conjugate is a protein native to the species of the human or mammal. In other embodiments, the protein or fragment thereof, is substantially homologous (i.e., at least 80%, 85%, 90%, 95%, 96%, 97%, 98%, or 99% identical in amino acid sequence over a length of at least 10, 25, 50, 100, 150, or 200 amino acids, or the entire length of the active agent) to a native sequence of the corresponding human or mammalian protein.

Methods for making recombinant proteins are well-known in the art. Methods of producing cells, including mammalian cells, which express DNA or RNA encoding a recombinant protein are described in U.S. patent numbers 6,048,729, 5,994,129, and 6,063,630.

In one embodiment, a nucleic acid construct used to express a polypeptide or fragment, or analog thereof is one which is expressed extrachromosomally (episomally) in the transfected mammalian cell or one which integrates, either randomly or at a preselected targeted site through homologous recombination, into the recipient cell's genome. A construct which is expressed extrachromosomally comprises, in addition to polypeptide-encoding sequences, sequences sufficient for expression of the protein in the cells and, optionally, for replication of the construct. It typically includes a promoter, a polypeptide-encoding DNA sequence and a polyadenylation site. The DNA encoding the protein is positioned in the construct in such a manner that its expression is under the control of the promoter. Optionally, the construct may contain additional components such as one or more of the following: a splice site, an enhancer sequence, a selectable marker gene under the control of an appropriate promoter, and an amplifiable marker gene under the control of an appropriate promoter.

In those embodiments in which the DNA construct integrates into the cell's genome, it includes the polypeptide-encoding nucleic acid sequences. Optionally, it can include a promoter and an enhancer sequence, a polyadenylation site or sites, a splice site or sites, nucleic acid sequences which encode a selectable marker or markers, nucleic acid sequences which encode an amplifiable marker and/or DNA homologous to genomic DNA in the recipient cell to target integration of the DNA to a selected site in the genome (targeting DNA or DNA sequences).

Host cells used to produce recombinant proteins are bacterial, yeast, insect, non-mammalian vertebrate, or mammalian cells; the mammalian cells include, but are not limited to, hamster, monkey, chimpanzee, dog, cat, bovine, porcine, mouse, rat, rabbit, sheep and human cells. The host cells include immortalized cells (a cell line) or non-immortalized (primary or secondary) cells and include any of a wide variety of cell types, such as, but not limited to, fibroblasts, keratinocytes, epithelial cells (e.g., mammary epithelial cells, intestinal epithelial cells), ovary cells (e.g., Chinese hamster ovary or CHO cells), endothelial cells, glial cells, neural cells, formed elements of the blood (e.g., lymphocytes, bone marrow cells), muscle cells, hepatocytes and precursors of these somatic cell types.

Commonly used host cells include prokaryotic cells such as gram negative or gram positive bacteria, *i.e.,* any strain of *E. coli, Bacillus, Streptomyces, Saccharomyces, Salmonella,* and the like; eukaryotic cells such as CHO (Chinese hamster ovary) cells; baby hamster kidney (BHK) cells; human kidney 293 cells; COS-7 cells; insect cells such as D. Mel-2, Sf4, Sf5, Sf9, and Sf21 and High 5; plant cells and various yeast cells such as *Saccharomyces* and *Pichia.*

Host cells containing the polypeptide-encoding DNA or RNA are cultured under conditions appropriate for growth of the cells and expression of the DNA or RNA. Those cells which express the polypeptide can be identified, using known methods, and the recombinant protein isolated and purified, using known methods; either with or without amplification of polypeptide production. Identification can be carried out, for example, through screening genetically modified mammalian cells displaying a phenotype indicative of the presence of DNA or RNA encoding the protein, such as PCR screening, screening by Southern blot analysis, or screening for the expression of the protein. Selection of cells having incorporated protein-encoding DNA may be accomplished by including a selectable marker in the DNA construct and culturing transfected or infected cells containing a selectable marker gene under conditions appropriate for survival of only those cells that express the selectable marker gene. Further amplification of the introduced DNA construct can be affected by culturing genetically modified cells under conditions appropriate for amplification (*e.g.,* culturing genetically modified cells containing an amplifiable marker gene in the presence of a concentration of a drug at which only cells containing multiple copies of the amplifiable marker gene can survive).

Recombinant proteins which are physiologically active proteins or therapeutic proteins include, but are not limited to, cytokines, growth factors, therapeutic coagulation proteins or blood clotting factors, enzymes, chemokines, soluble cell-surface receptors, cell adhesion molecules, antibodies, hormones, cytoskeletal proteins, matrix proteins, chaperone proteins, structural proteins, metabolic proteins, and other therapeutic proteins known to those of skill in the art. Exemplary recombinant proteins which are used as therapeutics include, but are not limited to, Factor VIII, Factor VII, Factor IX and von Willebrand factor, erythropoietin, interferons, insulin, CTLA4-Ig, alpha-glucocerebrosidase, alpha-glucosidase, follicle stimulating hormone, anti-CD20 antibody, anti-HER2 antibody, anti-CD52 antibody, TNF receptor, and others known in the art. See, for example, Physicians Desk Reference, 62nd Edition, 2008, Thomson Healthcare, Montvale, NJ.

In one embodiment, the protein is a therapeutic coagulation factor or blood (clotting) factor, including but not limited to, Factor II, Factor III, Factor V, Factor VIII, Factor VIII, Factor IX , Factor X, Factor XI, von Willebrand Factor and fibrinogen. In a related embodiment, the protein complex comprises one or more blood factors.

In one embodiment, the protein is plasma-derived and/or recombinant von Willebrand factor (VWF) or a biologically active fragment or analog thereof. The term "plasma-derived VWF (pVWF)" includes mature VWF obtained from a mammal. One biologically active fragment or analog of said pVWF is pro-VWF which contains the pro-peptide. In one example of the present invention, the protein is selected from the group consisting of immature VWF including the precursor VWF molecule (pre-pro-VWF) synthesized by endothelial cells and megakaryocytes, the VWF propeptide (pro-VWF), and mature plasmatic VWF obtained upon cleavage of the signal peptide and pro-peptide, respectively, of the precursor molecule. Further examples of biologically active derivatives of plasma-derived VWF include pro-drugs which are processed or converted into the biologically active form, or are biologically active as such, truncated forms, forms having deletions, forms having substitutions, forms having additions other than pro-forms, fragments of the mature form, chimeric forms, and forms having post-translational modifications as compared to the natural form. The term "recombinant VWF (rVWF)" includes VWF obtained via recombinant DNA technology having optionally a glycosylation pattern which is pharmacologically acceptable. Specific examples thereof include VWF without A2 domain thus resistant to proteolysis (Lankhof et al., Thromb Haemost.;77:1008-1013,1997) and the VWF fragment from Val 449 to Asn 730 including the glycoprotein Ib-binding domain and binding sites for collagen and heparin (Pietu et al., Biochem Biophys Res Commun.; 164:1339-1347, 1989).

In a related embodiment, the protein is Factor VIII. Previous experiments demonstrated that conjugation of a physiologically acceptable polymer molecule to Factor VIII results in a Factor VIII protein being activatable by thrombin and having a substantially decreased antigenicity and immunoreactivity, and a substantially increased *in vivo* clearance rate in the bloodstream of a mammal (U.S. Patent 4,970,300). International patent application WO 94/15625 describes that conjugating Factor VIII to a physiologically acceptable polymer molecule improves the *in vivo* function of Factor VIII (i) by increasing its resistance to *in vivo* hydrolysis and thus prolonging its activity after administration, (ii) by significantly prolonging its circulating life *in vivo* over unmodified protein, and (iii) by increasing its absorption time into the blood stream. Further, improving the *in vivo* function of Factor IX by binding it to physiologically acceptable polymer molecules, in particular poly(ethylene glycol) ("PEG"), has been described in the international patent application WO 94/29370 A1. However, none of these documents disclose the number of polymer molecules linked to the Factor VIII protein.

### Polypeptide Variants and Analogs

Methods of the invention are useful to rapidly detect recombinant proteins in a sample, as well as fragments, analogs or variants of the recombinant protein, and further may be useful to detect naturally-occurring protein which may exist as fragments or allelic variants in vivo wherein glycosylation differences can be detected.

Methods for preparing polypeptide fragments, analogs or variants are well-known in the art. Fragments of a polypeptide are prepared using methods well known in the art, including enzymatic cleavage (e.g., trypsin, chymotrypsin) and also using recombinant means to generate a polypeptide fragment having a specific amino acid sequence. Fragments may be generated to comprise a ligand-binding domain, a receptor-binding domain, a dimerization or multimerization domain, or any other identifiable domain known in the art.

Methods of making polypeptide analogs are also well-known. Analogs may he substantially homologous or substantially identical to the naturally-occurring polypeptide from which the analog is derived, and analogs contemplated by the invention are those which retain at least some of the biological activity of the naturally-occurring polypeptide.

Substitution analogs typically exchange one amino acid of the wild-type for another at one or more sites within the protein, and may be designed to modulate one or more properties of the polypeptide, such as stability against proteolytic cleavage, without the loss of other functions or properties. Substitutions of this kind are generally conservative. By "conservative amino acid substitution" is meant substitution of an amino acid with an amino acid having a side chain of a similar chemical character. Similar amino acids for making conservative substitutions include those having an acidic side chain (glutamic acid, aspartic acid); a basic side chain (arginine, lysine, histidine); a polar amide side chain (glutamine, asparagine); a hydrophobic, aliphatic side chain (leucine, isoleucine, valine, alanine, glycine); an aromatic side chain (phenylalanine, tryptophan, tyrosine); a small side chain (glycine, alanine, serine, threonine, methionine); or an aliphatic hydroxyl side chain (serine, threonine).

Polynucleotide analogs and fragments may be readily generated by a worker of skill to encode biologically active fragments, variants, or mutants of the naturally occurring molecule that possess the same or similar biological activity to the naturally occurring molecule. Routinely practiced methods include PCR techniques, enzymatic digestion of DNA encoding the protein molecule and ligation to heterologous polynucleotide sequences, and the like. For example, point mutagenesis, using PCR and other techniques well-known in the art, may be employed to identify with particularity which amino acid residues are important in particular activities associated with protein activity. Thus, one of skill in the art will be able to generate single base changes in the DNA strand to result in an altered codon and a missense mutation.

It is further contemplated that the protein or polypeptide may be modified to make an analog which is a fusion protein comprising a second agent which is a polypeptide. In one embodiment, the second agent which is a polypeptide is an enzyme, a growth factor, a cytokine, a chemokine, a cell-surface receptor, the extracellular domain of a cell surface receptor, a cell adhesion molecule, or fragment or active domain of a protein described above or of any other type of protein known in the art. In a related embodiment, the second agent is a blood clotting factor such as Factor VIII, Factor VII, Factor IX and von Willebrand factor. The fusion protein contemplated is made by chemical or recombinant techniques well-known in the art.

Protein variants contemplated include polypeptides chemically modified by such techniques as ubiquitination, glycosylation, conjugation to therapeutic or diagnostic agents, labeling (*e.g.,* with radionuclides or various enzymes), covalent polymer attachment such as PEGylation (derivatization with polyethylene glycol), introduction of non-hydrolyzable bonds, and insertion or substitution by chemical synthesis of amino acids such as ornithine, which do not normally occur in human proteins. Variants retain the binding properties of non-modified molecules of the invention.

Additional polypeptide variants useful in the methods of the present invention include polypeptides comprising polysialylate (PSA) moieties. Methods for preparing polysialylated polypeptide are described in U.S. Patent Publication 20060160948 and Saenko et al., Haemophilia 12:42-51, 2006.

### Polymers

In one embodiment, the invention contemplates chemically modified proteins or polypeptides, which have been linked to a chemical moiety that provides advantageous effects to production, viability of the protein or polypeptide. For example, nonspecific or site-specific conjugation of water-soluble polymers to polypeptides is known in the art to improve half-life by potentially reducing immunogenicity, renal clearance, and/or improving protease resistance.

Water-soluble polymers, including but not limited to, poly(ethylene glycol) (PEG), poly(ethylene oxide) (PEO), polyoxyethylene (POE), polyvinyl alcohols, hydroxyethyl celluloses, or dextrans, are commonly conjugated to proteins or peptides to increase stability or size, etc., of a protein or peptide.

PEG, PEO or POE refers to an oligomer or polymer of ethylene oxide. PEGs and PEOs include molecules with a distribution of molecular weights, i.e., polydisperse. The size distribution can be characterized statistically by its weight average molecular weight (Mw) and its number average molecular weight (Mn), the ratio of which is called the polydispersity index (Mw/Mn). Mw and Mn can be measured by mass spectroscopy. Most of the PEG-protein conjugates, particularly those conjugated to PEG larger than 1 KD, exhibit a range of molecular weights due to a polydisperse nature of the parent PEG molecule. For example, in case of mPEG2K (Sunbright ME-020HS, NOF), actual molecular masses are distributed over a range of 1.5 ~ 3.0 KD with a polydispersity index of 1.036. Exceptions are proteins conjugated to MS(PEG)n (N=4, 8, 12 or 24, e.g., PEO4, PEO12)-based reagents (Pierce), which are specially prepared as monodisperse mixtures with discrete chain length and defined molecular weight.

The invention contemplates use of water-soluble polymers that vary in type, conjugation, linkage and length. For example, PEG-protein conjugates include but are not limited to linear or branched conjugates, polymer:proteins conjugated by NHS (N-hydroxysuccinimide)- or aldehyde-based chemistry, variants with a different chemical linkage between the PEG chain and conjugation site, and variants differing in lengths. The average molecular weight of the PEG will range from about 3 kiloDalton ("kDa") to about 100 kDa, from about 5 kDa to about 60 kDa, from about 5 kDa to about 40 kDa, from about 5 kDa to about 25 kDa, from about 5 kDa to about 15 kDa, or from about 5 kDa to about 10 kDa.

The invention contemplates PEG-protein conjugates selected from the group consisting of linear PEG-protein conjugates that are NHS-conjugated and range in length from -(CH2-CH2-O)n-, where n = 1 to 2000, linear PEG-protein conjugates that are aldehyde-conjugated and range in length from-(CH2-CH2-O)n-, where n = 1 to 2000, two-arm branched PEG-protein conjugates that are NHS-conjugated and range in length, from 3 to 100 kDa in mass, and three-arm branched PEG-protein conjugates that are NHS-conjugated. The invention also contemplates PEG-protein conjugates that contain different chemical linkages (-CO(CH2)n-, and -(CH2)n-where n = 1 to 5) between its conjugation site and the PEG chain. The invention further contemplates charged, anionic PEG-protein conjugates to reduce renal clearance, including but not limited to carboxylated, sulfated and phosphorylated compounds (anionic) (Caliceti & Veronese, Adv Drug Deliv Rev 2003 55(10):1261-77; Perlman et al., J Clin Endo Metab 2003 88(7):3227-35; Pitkin et al., Antimicrob Agents Chemother 1986 29(3): 440-44; Vehaskari et al., Kidney Intl 1982 22 127-135). In a further embodiment, the peptide is optionally conjugated to a moiety including a bisphosphonate, a water-soluble polymer such as PEG or PEO, carbohydrates, fatty acids, or further amino acids.

Macromolecule chemical modification can be performed in a non-specific fashion (leading to mixtures of modified species) or in a site-specific fashion (based on wild-type macromolecule reactivity-directed modification and/or site-selective modification using a combination of site-directed mutagenesis and chemical modification) or, alternatively, using expressed protein ligation methods (Curr Opin Biotechnol. 13(4):297-303 (2002)).

To discover if the *in vivo* therapeutic half-life of a peptide would benefit from PEGylation, a variety of different PEG-protein conjugates are synthesized, characterized *in vitro* and in-vivo for pharmacokinetics. In order to both optimize the potential effects of PEGylation a design strategy is employed wherein polymer length, conformation, and charge of PEG is varied.

Methods for preparing the PEGylated protein of the present invention generally comprise the steps of (a) reacting the protein of interest with polyethylene glycol under conditions whereby PEG becomes attached to the N-terminus/C-terminus of the protein, and (b) obtaining the reaction product(s). Because PEGylating a protein might significantly alter the intrinsic activity of the protein, different types of PEG are explored. The chemistry that can be used for PEGylation of protein includes the acylation of the primary amines of the protein using the NHS-ester of methoxy-PEG (O-[(N-Succinimidyloxycarbonyl)-methyl]-O'-methylpolyethylene glycol). Acylation with methoxy-PEG-NHS or methoxy-PEG-SPA results in an amide linkage that eliminates the charge from the original primary amine (also, Boc-PEG for C-terminus). Unlike ribosome protein synthesis, synthetic peptide synthesis proceeds from the C-terminus to the N-terminus. Therefore, Boc-PEG is one method (i.e. using tert-(B)utyl (o)xy (c)arbonyl (Boc, t-Boc) synthesis) to attach PEG to the C-terminus of the peptide (R. B. Merrifield (1963). "Solid Phase Peptide Synthesis. 1. The Synthesis of a Tetrapeptide". J. Am. Chem. Soc. 85 (14): 2149-2154). (F)luorenyl-(m)eth(o)xy-(c)arbonyl (FMOC) chemistry (Atherton, E.; Sheppard, R.C. (1989). Solid Phase peptide synthesis: a practical approach. Oxford, England: IRL Press.) is favored because it does not require the hazardous use of hydrofluoric acid to remove side-chain protecting groups. The present methods provide for a substantially homogenous mixture of polymer:protein conjugate. "Substantially homogenous" as used herein means that only polymer:protein conjugate molecules are observed. The polymer:protein conjugate has biological activity and the present "substantially homogenous" PEGylated protein preparations are those which are homogenous enough to display the advantages of a homogenous preparation, *e.g.,* ease in clinical application in predictability of lot to lot pharmacokinetics.

In a further embodiment, the polymer molecules contemplated for use in the PEGylation approaches described herein may be selected from among water-soluble polymers or a mixture thereof. The polymer may have a single reactive group, such as an active ester for acylation or an aldehyde for alkylation, so that the degree of polymerization may be controlled. The water soluble polymer, or mixture thereof if desired, may be selected from the group consisting of, for example, PEG, monomethoxy-PEG, PEO, dextran, poly-(N-vinyl pyrrolidone), propylene glycol homopolymers, fatty acids, a polypropylene oxide/ethylene oxide co-polymer, polyoxyethylated polyols (*e.g*., glycerol), HPMA, FLEXIMAR™, and polyvinyl alcohol, mono-(C1-C10)alkoxy-PEG, aryloxy-PEG, tresyl monomethoxy PEG, PEG propionaldehyde, bis-succinimidyl carbonate PEG, cellulose, other carbohydrate-based polymers, or mixtures thereof. The polymer selected should be water-soluble so that the protein to which it is attached does not precipitate in an aqueous environment, such as a physiological environment. The polymer may be branched or unbranched. Preferably, for therapeutic use of the end-product preparation, the polymer will be pharmaceutically acceptable. Methods for generating peptides comprising a PEG moiety are well-known in the art. See, for example, US Patent 5,824,784.

In one embodiment, the reactive aldehyde is PEG- propionaldehyde, which is water-stable, or mono-C1-C10 alkoxy or aryloxy derivatives thereof (see U.S. Patent No. 5,252,714). As used herein, PEG is meant to encompass any of the forms of PEG that have been used to derivatize other proteins, such as mono-(C1-C10) alkoxy- or aryloxy-polyethylene glycol. The polymer may be branched or unbranched. Preferably, for therapeutic use of the end-product preparation, the polymer will be pharmaceutically acceptable.

### Pharmaceutical Compositions

The present invention contemplates pharmaceutical compositions comprising effective amounts of protein or derivative products of the invention together with pharmaceutically acceptable diluents, stabilizers, preservatives, solubilizers, emulsifiers, adjuvants and/or carriers. Such compositions include diluents of various buffer content (*e.g.,* Tris-HCl, phosphate), pH and ionic strength; additives such as detergents and solubilizing agents (*e.g.,* Polysorbate 20, Polysorbate 80), anti-oxidants (*e.g.,* ascorbic acid, sodium metabisulfite), preservatives (*e.g.,* Thimerosol, benzyl alcohol) and bulking substances (*e.g.,* lactose, mannitol); see, *e.g.,* Remington's Pharmaceutical Sciences, 18th Edition (1990, Mack Publishing Co., Easton, Pa.) pages 1435:1712. An effective amount of active ingredient is a therapeutically, prophylactically, or diagnostically effective amount, which can be readily determined by a person skilled in the art by taking into consideration such factors as body weight, age, and therapeutic goal.

The polymer-protein compositions of the present invention may also include a buffering agent to maintain the pH of the solution within a desired range. Preferred agents include sodium acetate, sodium phosphate, and sodium citrate. Mixtures of these buffering agents may also be used. The amount of buffering agent useful in the composition depends largely on the particular buffer used and the pH of the solution. For example, acetate is a more efficient buffer at pH 5 than pH 6 so less acetate may be used in a solution at pH 5 than at pH 6. The preferred pH range for the compositions of the present invention is pH 3.0-7.5.

The compositions of the present invention may further include an isotonicity-adjusting agent to render the solution isotonic and more compatible for injection. The most preferred agent is sodium chloride within a concentration range of 0-1500 mM.

The methods described herein use pharmaceutical compositions comprising the molecules described above, together with one or more pharmaceutically acceptable excipients or vehicles, and optionally other therapeutic and/or prophylactic ingredients. Such excipients include liquids such as water, saline, glycerol, polyethylene glycol, hyaluronic acid, ethanol, cyclodextrins, modified cyclodextrins (*i.e.,* sulfobutyl ether cyclodextrins), *etc.* Suitable excipients for non-liquid formulations are also known to those of skill in the art.

Pharmaceutically acceptable salts can be used in the compositions of the present invention and include, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients and salts is available in Remington's Pharmaceutical Sciences, 18th Edition (Easton, Pennsylvania: Mack Publishing Company, 1990).

Additionally, auxiliary substances, such as wetting or emulsifying agents, biological buffering substances, surfactants, and the like, may be present in such vehicles. A biological buffer can be virtually any solution which is pharmacologically acceptable and which provides the formulation with the desired pH, *i.e.,* a pH in the physiologically acceptable range. Examples of buffer solutions include saline, phosphate buffered saline, Tris buffered saline, Hank's buffered saline, and the like.

### Kits

Kits are also contemplated. A typical kit can comprise protein-polymer complex having a standard number of polymers attached. In one embodiment the kit further comprises a binding agent, which specifically binds the protein or polymer, wherein the binding agent is an antibody, a soluble receptor, a ligand, a cofactor or another agent that specifically binds the protein or polymer. The kit may optionally include reagents and buffers for preparation of the samples for detection of the polymer-protein complex.

Additional aspects and details of the invention will be apparent from the following examples, which are intended to be illustrative rather than limiting.

### Examples

### Example 1 PEGylation of Human Serum Albumin

In order to measure the degree of water soluble polymer on a protein using NIR, a protein of known molecular weight was conjugated to a PEG of a known size. Human Serum Albumin was PEGylated via lysine residues using linear PEG Succinimidyl succinate (PEG-SS / chain length: 5 kDa) (SunBio Inc., Anyang City, South Korea). A solution of HSA (concentration: 10 mg/ml) in 25 mM sodium acetate buffer, pH 6.2 was prepared and PEG-SS was added to give a final concentration of 120 mg reagent/mg protein. The mixture was incubated for 1 hour at room temperature under gentle shaking. After a reaction time of 10 minutes the pH was adjusted to pH 6.2 by drop-wise addition of 0.5 M NaOH. Subsequently the conjugate was purified by anion-exchange chromatography using a linear flow rate of 1 cm/min. 200 ml of the reaction mixture was applied to a Pharmacia XK26 column (26 mm x 155 mm) filled with DEAE-Sepharose FF (GE Healthcare, Waukesha, WI) and the column was equilibrated with 10 column volumes (CV) starting buffer (25 mM sodium acetate, pH 6.2). The PEG-HSA conjugate was eluted from the column with 25 mM sodium acetate buffer, pH 4.5 and the OD at 280 nm was measured to determine protein concentration.

### Example 2 Preparation of HSA species with different PEGylation degree

For preparation of HSA species with different PEGylation degree Human Serum Albumin is PEGylated via lysine residues as described in Example 1 using linear PEG Succinimidyl succinate (chain length: 5 kDa) and different amounts of reagent. A solution of HSA (concentration: 10 mg/ml) in 25 mM sodium acetate buffer, pH 6.2 is prepared and PEG-SS is added to give final concentrations of 30, 60, 90 and 120 mg reagent/mg protein. Subsequently the conjugates are purified by anion-exchange chromatography on DEAE-Sepharose FF. The PEG-HSA conjugates are eluted from the column with 25 mM sodium acetate buffer, pH 4.5 and the OD at 280 nm are measured to determine the protein concentration. Finally the PEGylation degree is estimated by the colorimetric method according to Nag et al. (Anal Biochem 250:35-43, 1997) and expressed in mol PEG/mol protein. For the different HSA preparations PEGylation degrees with 2, 3, 4, and 5 mole PEG / mol protein (amounts of reagent: 30, 60, 90, 120 mg / mg protein) are determined. These results are confirmed by measuring the PEGylation degree using the NIRVIS method according to Examples 3 and 4 below.

### Example 3 Process control of PEGylation

One strength of NIR-spectrometry lies in the possibility for control of the attachment of the polymer conjugation reaction and determining the amount of polymer on the conjugated molecule. The following example demonstrates the application of NIR to analyze the PEGylation of a sample of human serum albumin.

NIR spectras were collected continuously in the transflection mode during the entire reaction time of approximately one hour of the PEGylation in aqueous solution (as described above) with a standard NIR spectrometer in the spectral range from 4008 cm⁻¹ to 9996 cm⁻¹ (2500 nm to 1000 nm). Transflection measures transmission of light through a sample, wherein the light then contacts a reflector behind the sample, to allow the incoming light to be transmitted through the sample twice.

From these spectra sets, an adequate number of spectras before PEG addition (value = 0) and at the end of the reaction (value = 1) was selected for computing a calibration curve by the chemometric software of the spectrometer using a multiplicative scatter correction (MSC) as data pretreatment and a partial least squares (PLS)-algorithm as commonly known in the art.

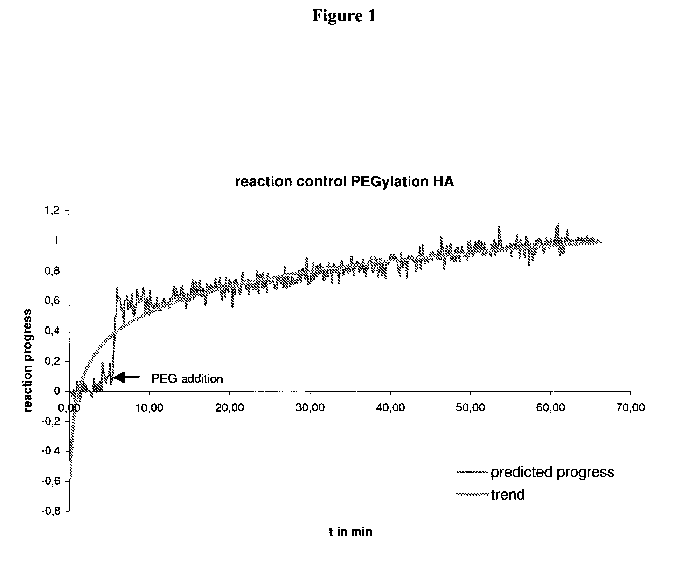

This calibration model was used to predict the remaining spectra collected during the reaction. As shown in Figure 1, a clear logarithmic trend after addition of the PEG-reagent is obtained.

These experiments demonstrate that the creation of calibration models for the prediction of the PEGylation progress during a PEGylation reaction is possible using NIR spectroscopy.

### Example 4 Chemometric measurement of PEGylation

In order to demonstrate the ability of the NIR-method to measure protein-bound PEG, a dilution series was prepared using a PEGylated HSA solution obtained by use of the conditions of Example 2. The degree of PEGylation was determined according to the method of Nag et al. (Anal Biochem 250:35-43, 1997) and was confirmed by mass spectrometry. These dilutions were analyzed by NIR and a representative number of near infrared spectras were collected by a standard NIR-spectrometer. The spectras were measured in the transflection mode in the spectral range from 4008 cm⁻¹ to 9996 cm⁻¹ (2500 nm to 1000 nm).

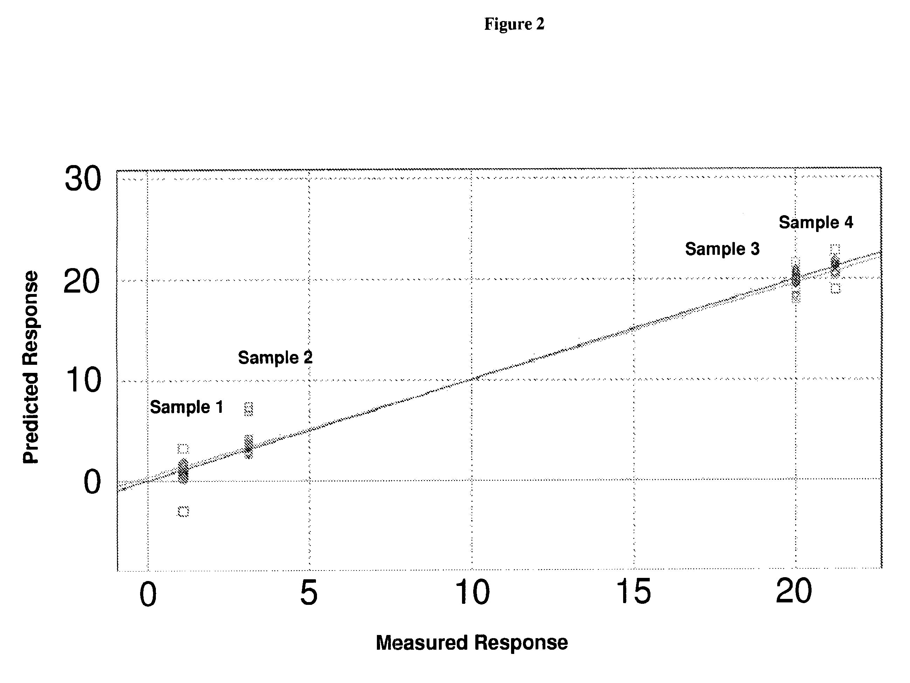

From these data collected, a calibration model was computed by the chemometric software of the spectrometer using the first derivative BCAP as data pretreatment and a PLS-algorithm. The results of these calculations are shown in Figure 2.

As shown in the results, a strong correlation between the "Measured Response" of PEG bound and the "Predicted Response" of PEG-bound exists. The Pearson correlation coefficient amounts to 0.996 with a P-value of 0.000. In the lower concentration range (solution 1 and 2, see Figure 2) an average deviation of 17% and 6% of the predicted value of PEG molecules to the true value of PEG molecules was achieved. In the higher concentration range (solution 3 and 4, see Figure 2) the average deviation from the true value was lower, 0.8 % for solution 3 and 1.4% for solution 4.

Theses results demonstrate that the measured responses obtained by the NIR method correlate well with the levels of protein-bound PEG in the sample. This implies that the method is able to predict the degree of PEGylation on a protein molecule and is useful to determine the number and degree of PEGylation and other polymer conjugation on a protein molecule.

Numerous modifications and variations in the invention as set forth in the above illustrative examples are expected to occur to those skilled in the art. Consequently only such limitations as appear in the appended claims should be placed on the invention.

## Claims

1. A method for determining a number of water soluble polymer moieties conjugated to a protein or protein complex in a sample, comprising contacting the sample with a light source in the near infrared spectrum and measuring relative absorbance levels over a range of the near-infrared wavelengths,
determining the number of polymer molecules conjugated to the protein or protein complex based on wavelength or wavenumber spectra, wherein the spectra of the sample is compared to a previously calculated standard having a known amount of polymer molecules conjugated to a protein or protein complex, wherein the near infrared wavelength is in the range of 680 nm to 2500 nm.

2. The method of claim 1 wherein the contacting further comprises measuring reflected radiation from said sample as a plurality of time-dependant absorbance spectra.

3. The method of claim 1 wherein the comparison comprises constructing a calibration data matrix from a plurality of near-infrared absorption spectra of known samples and comparing the measured spectroscopy of the sample comprising the polymer-protein conjugate.

4. The method of claim 1 wherein the near infrared wavelength is in the range of from 680 nm to 1100 nm; or from 1100 nm to 2500 nm; or from 1000 nm to 2500 nm.

5. The method of claim. 1 wherein the water soluble polymer is polyethylene glycol (PEG).

6. The method of claim 5 wherein the PEG has a molecular weight in a range of 3 kDa to 100 kDa.

7. The method of claim 5 wherein the PEG has a molecular weight in a range of 5 kDa to 60 kDa.

8. The method of claim 5 wherein the PEG has a molecular weight in a range of 5 kDa to 40 kDa.

9. The method of claim 5 wherein the PEG has a molecular weight in a range of 5 k Da to 15 kDa.

10. The method of claim 5 wherein the PEG has a molecular weight in a range of 5 kDa to 10 kDa.

11. The method of claim 1 wherein the protein or protein complex is a therapeutic coagulation protein or a therapeutic coagulation protein complex.

12. The method of claim 1 wherein the protein or protein complex comprises a blood factor selected from the group consisting of Factor II, Factor III, Factor V, Factor VII, Factor VIII, Factor IX, Factor X, Factor XI, von Willebrand Factor and fibrinogen.

13. The method of claim 12 wherein the protein is von Willebrand factor.

14. The method of claim 12 wherein the protein complex comprises von Willebrand Factor and Factor VIII.

15. A process for making a composition having a uniform number of water soluble polymer molecules on a protein or protein complex comprising:
conjugating the protein to a water soluble molecule to produce a sample of a polymer-protein complex;
performing the method of claim 1 on the polymer-protein complex; and isolating compounds within the sample having the same number of polymer molecules based on the near infrared absorbance spectra.

## Patentansprüche

1. Verfahren zum Bestimmen einer Anzahl von wasserlöslichen Polymereinheiten, die an ein Protein oder einen Proteinkomplex konjugiert sind, in einer Probe, umfassend das Inkontaktbringen der Probe mit einer Lichtquelle im Nahinfrarotspektrum und das Messen relativer Niveaus des Absorptionsvermögens über einen Bereich der Nahinfrarotwellenlängen,
Bestimmen der Anzahl von Polymermolekülen, die an das Protein oder den Proteinkomplex konjugiert sind, basierend auf Wellenlängen- oder Wellenzahlspektren, wobei die Spektren der Probe mit einem zuvor berechneten Standard verglichen werden, welcher eine bekannte Menge von Polymermolekülen, die an ein Protein oder einen Proteinkomplex konjugiert sind, aufweist, wobei die Nahinfrarotwellenlänge im Bereich von 680 nm bis 2500 nm liegt.

2. Verfahren nach Anspruch 1, wobei das Inkontaktbringen weiter das Messen reflektierter Strahlung von der Probe als eine Vielzahl von zeitabhängigen Absorptionsvermögensspektren umfaßt.

3. Verfahren nach Anspruch 1, wobei der Vergleich das Erstellen einer Kalibrationsdatenmatrix aus einer Vielzahl von Nahinfrarotabsorptionsspektren bekannter Proben und das Vergleichen der gemessenen Spektroskopie der Probe, welche das Polymer-Proteinkonjugat aufweist, umfaßt.

4. Verfahren nach Anspruch 1, wobei die Nahinfrarotwellenlänge im Bereich von 680 nm bis 1100 nm, oder von 1100 nm bis 2500 nm, oder von 1000 nm bis 2500 nm liegt.

5. Verfahren nach Anspruch 1, wobei das wasserlösliche Polymer Polyethylenglycol (PEG) ist.

6. Verfahren nach Anspruch 5, wobei das PEG ein Molekulargewicht im Bereich von 3 kDa bis 100 kDa aufweist.

7. Verfahren nach Anspruch 5, wobei das PEG ein Molekulargewicht im Bereich von 5 kDa bis 60 kDa aufweist.

8. Verfahren nach Anspruch 5, wobei das PEG ein Molekulargewicht im Bereich von 5 kDa bis 40 kDa aufweist.

9. Verfahren nach Anspruch 5, wobei das PEG ein Molekulargewicht im Bereich von 5 kDa bis 15 kDa aufweist.

10. Verfahren nach Anspruch 5, wobei das PEG ein Molekulargewicht im Bereich von 5 kDa bis 10 kDa aufweist.

11. Verfahren nach Anspruch 1, wobei das Protein oder der Proteinkomplex ein therapeutisches Koagulationsprotein oder ein therapeutischer Koagulationsproteinkomplex ist.

12. Verfahren nach Anspruch 1, wobei das Protein oder der Proteinkomplex einen Blutfaktor umfaßt, ausgewählt aus der Gruppe, bestehend aus Faktor II, Faktor III, Faktor V, Faktor VII, Faktor VIII, Faktor IX, Factor X, Faktor XI, von Willebrand-Faktor und Fibrinogen.

13. Verfahren nach Anspruch 12, wobei das Protein von Willebrand-Faktor ist.

14. Verfahren nach Anspruch 12, wobei der Proteinkomplex von Willebrand-Faktor und Faktor VIII umfaßt.

15. Verfahren zum Herstellen einer Zusammensetzung, die eine gleichförmige Anzahl von wasserlöslichen Polymermolekülen auf einem Protein oder Proteinkomplex aufweist, umfassend:
Konjugieren des Proteins an ein wasserlösliches Molekül, um eine Probe eines Polymer-Proteinkomplexes zu erzeugen,
Durchführen des Verfahrens nach Anspruch 1 mit dem Polymer-Proteinkomplex, und Isolieren von Verbindungen innerhalb der Probe, welche die gleiche Anzahl von Polymermolekülen aufweist, basierend auf den Nahinfrarotabsorptionsvermögensspektren.

## Revendications

1. Procédé de détermination d'un nombre de fractions de polymères solubles dans l'eau, conjuguées à une protéine ou à un complexe de protéine dans un échantillon, comprenant la mise en contact de l'échantillon avec une source de lumière dans le spectre du proche infrarouge et la mesure de niveaux d'absorption relatifs sur une plage des longueurs d'onde du proche infrarouge,
la détermination du nombre de molécules de polymère conjuguées à la protéine ou au complexe de protéine sur la base de spectres de longueur d'onde ou de nombre d'onde, dans lequel les spectres de l'échantillon sont comparés à un standard calculé préalablement, comportant une quantité connue de molécules de polymère conjuguées à une protéine ou à un complexe de protéine, dans lequel la longueur d'onde du proche infrarouge est dans la plage de 680 nm à 2500 nm.

2. Procédé selon la revendication 1, dans lequel la mise en contact comprend en outre la mesure de rayonnement réfléchi à partir dudit échantillon sous la forme d'une pluralité de spectres d'absorption en fonction du temps.

3. Procédé selon la revendication 1, dans lequel la comparaison comprend la construction d'une matrice de données d'étalonnage à partir d'une pluralité de spectres d'absorption du proche infrarouge d'échantillons connus et la comparaison de la spectroscopie mesurée de l'échantillon comprenant le conjugué polymère-protéine.

4. Procédé selon la revendication 1, dans lequel la longueur d'onde du proche infrarouge est comprise dans la plage de 680 nm à 1100 nm ; ou de 1100 nm à 2500 nm ; ou de 1000 nm à 2500 nm.

5. Procédé selon la revendication 1, dans lequel le polymère soluble dans l'eau est du polyéthylène glycol (PEG).

6. Procédé selon la revendication 5, dans lequel le PEG présente un poids moléculaire dans une plage de 3 kDa à 100 kDa.

7. Procédé selon la revendication 5, dans lequel le PEG présente un poids moléculaire dans une plage de 5 kDa à 60 kDa.

8. Procédé selon la revendication 5, dans lequel le PEG présente un poids moléculaire dans une plage de 5 kDa à 40 kDa.

9. Procédé selon la revendication 5, dans lequel le PEG présente un poids moléculaire dans une plage de 5 kDa à 15 kDa.

10. Procédé selon la revendication 5, dans lequel le PEG présente un poids moléculaire dans une plage de 5 kDa à 10 kDa.

11. Procédé selon la revendication 1, dans lequel la protéine ou le complexe de protéine est une protéine de coagulation thérapeutique ou un complexe de protéine de coagulation thérapeutique.

12. Procédé selon la revendication 1, dans lequel la protéine ou le complexe de protéine comprend un facteur sanguin sélectionné à partir du groupe constitué par le facteur II, le facteur III, le facteur V, le facteur VII, le facteur VIII, le facteur IX, le facteur X, le facteur XI, le facteur de von Willebrand et le fibrinogène.

13. Procédé selon la revendication 12, dans lequel la protéine est le facteur de von Willebrand.

14. Procédé selon la revendication 12, dans lequel le complexe de protéine comprend le facteur de von Willebrand et le facteur VIII.

15. Procédé de réalisation d'une composition comportant un nombre uniforme de molécules de polymère solubles dans l'eau sur une protéine ou un complexe de protéine comprenant :
la conjugaison de la protéine à une molécule soluble dans l'eau afin de produire un échantillon d'un complexe polymère-protéine ;
la mise en oeuvre du procédé selon la revendication 1 sur le complexe polymère-protéine ; et l'isolation des composés à l'intérieur de l'échantillon comportant le même nombre de molécules de polymère sur la base des spectres d'absorption en proche infrarouge.
